# EUROPEAN PATENT APPLICATION

(11) **EP 3 922 250 A1**
(43) Date of publication of application: **15.12.2021**
(21) Application number: 20867507.4
(22) Date of filing: 15.06.2020
(51) Int. Cl.: A61K 31/496, A61K 9/08, A61K 9/20, A61P 25/20

(54) **COMPOSITION FOR CORRECTING THE BEHAVIOUR OF CATS AND DOGS**

(30) Priority: 24.09.2019 RU 2019129981
(71) Applicant: Obshestvo S Ogranichennoy Otvetstvennost'Yu "Nauchno-Proizvodstvennaya Kompaniya "Skiff", Moscow, 117246 (RU)
(72) Inventor: ZEYNALOV, Orkhan Akhmed ogly, Moscow, 117218 (RU); ZOLOTAREVA, Vera Anatol'evna, Krasnogorsk Moskovskaya Obl., 143405 (RU)
(74) Representative: Zellentin & Partner mbB Patentanwälte
(86) International application number: PCT/RU2020/000284
(87) International publication number: WO 2021/061006

(57) **Abstract**

The invention relates to the pharmaceutical industry and veterinary medicine, and constitutes a pharmaceutical composition for correcting the behaviour of cats and dogs in stressful situations without causing lethargy and/or loss of coordination of movement and/or prolonged deep sleep, characterised in that it comprises trazodone succinate as an active ingredient in a concentration that provides an effective dose in a single administration, and pharmaceutically acceptable fillers.

## Description

### Technical Field

The invention relates to pharmaceuticals and is intended for veterinary use to address commonly occurring behavioral issues related to stress conditions in cats and dogs. There is provided a sedative (anti-anxiety, anti-stress) product effective for modifying behavior in small domestic animals showing fear (anxiety) of transportation, loud sounds, visits to new locations, separation from their owners, or in other common life situations. The novel product contains, as an active principle, Trazodone Succinate which, while showing the same sedative efficacy as Trazodone or Trazodone Hydrochloride traditionally used in medicine, eliminates the side effects specific to Trazodone-containing pharmaceuticals or at least significantly reduces their severity.

According to expert estimates, up to 90% of dogs living at home may have certain behavioral anomalies, both those specific to their breed and/or those of psychological type that are exhibited permanently or in certain stressful situations. The permanent behavioral deviations include: aggressiveness to their fellows and humans, excessive vocalizing and motion activity, teeth showing, etc.; typical situational anomalies include various phobias or anxieties related to loud sounds (thunderstorm, fireworks), separation from their owners, transportation, visits to veterinary clinics. The latter are also common to many domestic cats. Of course, such abnormal behaviors in dogs and cats put their owners to trouble and urge them to search for reliable methods and means to address the problem in a reasonably short time and to modify their pets' behavior. One of such methods is psychological preparation of animals through special training practices, which, however, is time consuming, while not always effective. There is, therefore, still a need for the development of veterinary products having a sedative (anti-stress) effect for small domestic animals.

### Background

Information on veterinary products with anxiolytic or psychotropic effect intended specifically for modifying abnormal behavior in small domestic animals in stressful situations is extremely limited, as well as the number of such products.

One group of sedatives for small domestic animals include products which are based on the use of pheromones, such as, for example, a dog behavior modifier spray Adaptil manufactured by a French company CEVA SANTE ANIMALE and a similar product Feliway (CEVA) which is a cat behavior modifier *(**http:*//*www.vetlek.ru*/*shop*/*?gid*=*32151&id*=*8789).*

Common disadvantages of the products of this type are short duration of their action, a narrow spectrum of activity which is actually limited to such stressful situations as putting or moving an animal to an unordinary or uncomfortable location, as well as high allergenicity and high price.

A second, the most numerous, group of sedatives for cats and dogs includes multi-agent compounds comprising various combinations of the known medicinal plants. Typical representatives of this group of sedatives are Fitex Sedative Drops (Gruppa EKSPA OOO, available at *http:*//*www.fauna-vesh.ru*/*catalog.php?id=242),* Kot Bayun (Veda OOO, available at *https:llveterinarka.ru*/*vetmedicaments*/*kot-bayun*), and the Relaxivet product line (EcoProm, available at *https:*//*www.ecoyrm.org*/*catalog*/*476).*

The main disadvantages of the products from this group are their low efficacy, requirement for a course treatment, and slow action that does not allow bringing an animal's behavior to normal in a short time.

Finally, there is a third group of products used in the veterinary treatment of small domestic animals for the above purposes, which includes relatively new products containing, as active ingredients, certain compounds that have been known in the medical field (psychiatry) for their anxiolytic and/or antidepressant effects, in particular, Phenibut (Patent RU No. 2367428 C1.20.09.2009) and Guaiphenesine (Patent RU No. 2683936, 03.04.2019). The former is a complex combination drug with a mild effect, comprising 6 main active ingredients and intended to be administered as a course starting several days prior to an anticipated stress and for several days after the end thereof, while the second, i.e. the Guaiphenesine, has a disadvantage of being unpalatable to animals (cats), with associated difficulties in administration and side effects.

Since relatively recently, some foreign researchers have included Trazodone, an (atypical) antidepressant used in psychiatry, into the group of tranquilizers considered as potential candidates for use to modify dog and cat behavior in stressful situations.

Trazodone (TR) was synthesized in mid-sixties in Italy and was granted patent protection (on the basis of the Italian Application 2780665 15.12.1965, IT) in the USA (US 3.381.009 30.04.1968) and several European countries (Great Britain - GB 117068, 12.06.68; Belgium - BE 743313, 28.05.70, etc.) as part of a group of 1,2,4-triazolo[4,3-a]pyridines with tranquilizing, analgesic, and hypotensive effects.

In the next 20 years, extensive researches were made to study the TR pharmacology and pharmacokinetics, as well as its antidepressant and anxiolytic properties (Bryant SG., Clin Pharm. 1982 Sep-Oct;1(5):406-17; Byrne JE., 1982.259(2):259-70; Fabre LF., J Clin Psychiatry. 1990 Sep;51 Suppl:23-6; Rickels K., Arch Gen Psychiatry. 1993 Nov;50(11):884-95, etc.).

It is currently known that, after ingestion, TR is rapidly adsorbed from the gastrointestinal tract; the time to reach maximum plasma concentration is 1 to 2 hours. The substance rapidly passes through histo-hematic barriers, including the hematoencephalic barrier, rapidly enters into tissues and body fluids (bile, saliva, breast milk), with an amount of plasma protein binding of 89-95%. TR is actively metabolized in the liver by hydroxylation and oxidation in the presence of cytochrome izo-enzymes p450 (CYP3A4, CYP3A5 and CYP3A7).

TR is excreted from the body (about 20% excreted unchanged with bile, while 75% is excreted from the body via the kidneys) in two phases (Bryant S., Clin Pharm. 1982, 1(5):406-17), where, based on various data, T1/2 is 1 to 5 hours for the first phase, 5 to 12 hours for the second phase, while the time to reach complete excretion of the substance is 98 hours.

Regarding the pharmaceutical properties of the TR, the main effect produced by this substance in humans is, according to expert estimates, the antidepressant effect. The TR has also been found to produce thymoleptic, anxiolytic, sedative and myorelaxing effects. The TR action mechanism is based on interactions with serotonin receptors in the brain, primarily with those of A and C 5-HT2 sub-types, being their antagonist, and on selectively inhibiting the process of serotonin reuptake by brain neuron synaptosomes (Marek G.J., et. al., 1992, Psychopharmacology, 109, 2-11).

Today, TR is registered in many countries as a medicinal product and is widely used in medical practice, primarily, in psychiatry. Indications for medicinal use thereof include psychic (affective tension, irritation, fear) and somatic (rapid heartbeat, headache, myalgia, frequent urination, hyperhidrosis) symptoms of anxiety; depression; various phobias; insomnia. Trazodone is used in the medical field in the form of oral tablets. Examples of Trazodone-based products in use are: Trittico (Aziende Chimiche Riunite ANGELINI FRANCESCO (A.C.R.A.F. S.p.A.)) - TR hydrochloride tablets; Desyrel (Pragma Pharms Lie.) - TR hydrochloride tablets; Oleptro (Angelini Pharma) - extended-release TR hydrochloride tablets. From the above products, the Trittico tablets are available in the RF, which are included into the National Register of Medicines Approved for Use.

Noteworthy is the fact that, while in many scientific research papers TR is reported as suitable for use both in its free state and as a pharmaceutically acceptable salt, in all currently known commercial formulations for medicinal use it is used only in the form of a chloride, i.e. Trazodone hydrochloride, and all experimental data from scientific and clinical studies are applicable to this particular salt, so no other TR salts' properties or specific features have been actually studied.

Based on its practical use in medicine, it is well known that Trazodone Hydrochloride produces such side effects as vomiting, diarrhea, drowsiness (pronounced soporific effect), dystaxia, atony, asthenia, and even faintness. In most reported cases, the main disadvantage is a pronounced sedative action (Fabre LF. 1990, J Clin Psychiatry.;51 Suppl:23-6). As such, many scientific development projects are currently aimed at eliminating the above adverse body effects from the TR.

Thus, under RU 2 126801 C1, (27.02.1999), legal protection is granted to a pharmaceutical composition having a Trazodone activity and reduced side effects, the composition comprising an active agent and a carrier, wherein the active agent is an alkyl (in particular, methyl-)-derivative of Trazodone or a pharmaceutically acceptable salt thereof; RU 2 128180 C1 (27.03.1999) discloses an (S)- or (R)-enantiomer of Trazodone which is alkyl-substituted at one position or a pharmaceutically acceptable salt thereof, which, while having pharmacological properties of Trazodone, have lower affinity for adrenergic receptors, thus eliminating the side effects related thereto; in EA 017019 B1 (28.09.2012) there are provided a method for purifying Trazodone and a pharmaceutical composition produced from the purified substance, the composition comprising Trazodone or Trazodone Hydrochloride containing not more than 15 parts per million of alkylating agents responsible for various side effects.

As of yet, no Trazodone-based medicinal product is available on the global veterinary market, which is intended specifically for animal use. However, professional community in some foreign countries is actively investigating the possibilities to use the existing Trazodone-containing medicinal products to modify abnormal behavior in cats and dogs, in particular, anxiety disorders including the fears of transportation, separation from the owner, loud sounds, visits to veterinary clinics and/or hospitalization, motion restrictions, e.g., during a post-surgery period, or in cases of aggressive behavior (Gruen ME., 2008, J Am Vet Med Assoc., 233(12): 1902-7; Gruen ME, 2014, J Am Vet Med Assoc., 245(3): 296-301; Gilbert-Gregory SE, J Am Vet Med Assoc., 249 (11): 1281-1291; Stevens B., 2016, J Am Vet Med Assoc., 249(2):202-7; Burma Chea and Mario Georgi., 2017, Am.J.Anim.Vet. Sci., 12(4), 188-194; US 5,554.383, 10.09.1996).

Based on the above studies, practicing veterinarians in some countries recommend to cat and dog owners to use, in various stressful situations, Trazodone directly in the form of currently known medicinal products (available at: *http:*//*www.dvm360.com:* John Ciribassi, 01.05.2015; Jennifer L. Garcia; 16. 02.2017; Kathryn Primm 27.06.2016; Karen L. Overall, 01.08.2014, etc.). In view of this, it should not come as a surprise that the numerous side effects inherent to the known commercially available products, such as, primarily, heavy sleep, atony, slowness, dystaxia, observed during several hours after administration of a Trazodone-containing product, are also observed when such products are used for cats and dogs.

However, where such products are administered to pets on an ad-hoc basis, an hours-long heavy sleep or a psychophysiological state disturbing the normal routine of the animal or its owner's life (during and/or after the stress factor occurrence), are, in many cases, highly undesirable side effects of the product.

Exceptional among stressful situations associated with undesirable behaviors, especially in male and female cats, is the heat period, since its underlying mechanisms are not directly related to the senses of fear or anxiety. Today, one of the main methods for modifying the behavior related to natural practicing by the cats of their reproductive function is the systematic use of hormonal contraceptive products (HCP), primarily, the state-of-the art bi-hormonal HCP producing a mild and reversible effect on animals (Zeinalov, O.A. et al., 2019, Russian Veterinarian Journal, No.3, 12-20). However, in certain situations, use of bi-hormonal contraceptives is contraindicated, such as, in particular, where more than 2 days have passed from the heat period start, or where a cat suffers from one of the diseases listed in such products' leaflets (ref., for example, to the SexBarier Product Leaflet available at: *http:*//*ww.skiff-pharm.ru*/*seks-baryer-dlya-koshek-kapli*).

In some of the above cases, veterinarians recommend using various psychopharmaceuticals that modify animal behavior (Simpson B.S., Simpson D.M. Behavioral pharmacotherapy. Part II. Anxiolytics and mood stabilizers // Compendium on Continuing Education for the Practicing Veterinarian-2006. - Vol. 18, P. 1067-1081; Overall K.L. Pharmacologic treatments for behavior problems // Veterinary Clinics of North America: Small Animal Practice - 2007.- Vol. 27, P. 637-665). While a course treatment is the key factor for such products to have effect, no products specifically intended for ad-hoc administration to rapidly address behavioral issues in cats and dogs during heat periods are currently available on the veterinary market.

### Summary of the Invention

It has been an objective of the present invention to develop a novel Trazodone-containing sedative product for modifying behavior in cats and dogs in various stressful situations, which would be free of side effects, such as atony, and/or dystaxia, and/or lengthy heavy sleep (the technical result).

The above objective has been accomplished by using, as an active principle, a specific Trazodone salt, i.e. Trazodone Succinate, in a sedative product for small domestic animals. The theoretical basis for the proposed solution was provided by prior art data on useful properties exerted by succinic acid in mammal bodies.

A human body daily produces approximately 20 mg of succinic acid; however, this amount is almost entirely consumed in the numerous metabolic processes wherein it is involved, such as, in particular, in one of the most important energy cycles, which is the Krebs cycle.

Succinic acid is known to promote energy metabolism in humans and agricultural animals, as well as to have pronounced antioxidant and cardioprotective effects (Bakankin, A.V., *Farmako-toksikologicheskoye obosnovaniye primeneniya yantarnoy kisloty v zhivotnovodstve i veterinarii (Pharmacological and Toxicological Justification for the Use of Succinic Acid in Animal Husbandry and Veterinary Medicine),* an author's abstract of dissertation, Kazan, 2007, available at: *http:*//*medical-diss.com*/*veterinariya*/*farmako-toksikologicheskoe-obosnovanie-primeneniya-yantarnoy-kisloty-v-zhivotnovodstve-i-veterinarii#ixzz5VjpDankP;* Gross GJ Mol Cell Biochem. 2005 Apr; 272(l-2):221-7; https://cosmetic-oil.com/?p=3951).

Furthermore, there is literature data on detoxification properties exerted by succinic acid in mammal bodies by increasing the rate of catabolism of toxic compounds (Krop S, Fed Proc. 1946;5 (1 Pt 2): 187) and on its immunoprotective properties exerted by enhancing the body's protective functions (https://adella.ru/health/yantamaya-kislota.html), as well as data on the anti-stress effect produced by the above compound in certain animal species, in particular, in poultry (Basankin, A.V., 2007). On the contrary, in many cases it has been observed that the level of intrinsic succinate synthesis by the body reduces in stressful situations, which also occurs in conditions that cause a "stressed" immune status (*https :*//*alko03.ru*/*opvanenie*/*pohmele*/*vantarnava-kislota.html*).

Of special importance is the succinates' capability to normalize sleep, and, as such, succinic acid is recommended, both in the official and the traditional medicine, as a remedy to control depression, chronic fatigue and drowsiness (Zhang JH et al., J. Ethnopharmacol. 2018, 213:230-255).

However, it is noteworthy that, to accomplish the objective of modifying small domestic animal behavior in stressful situations, the active ingredient (Trazodone salt) is to produce effect after a single dose administration, rapidly enough and at relatively low doses, while medicinal use of the succinate in the manner enabling implementation of the compound's useful properties provides for a long-term course treatment with the acid, wherein the acid is used at high concentrations. In view of this, when developing the present invention, it remained unclear whether the amount of the succinate anion produced as a result of the salt dissociation would be sufficient to eliminate the pronounced side effects of Trazodone. As such, accomplishment of the desired technical result, despite the fact that this compound possesses the above useful properties, in this case was not obvious to a person skilled in the art.

### In practical terms, development of the novel anthelmintic included the steps of:

### a) Producing Trazodone Succinate and a dosage form based thereon.

Trazodone Succinate was produced from Trazodone Hydrochloride (manufactured by Shenyang Funing Parmaceutical Co.Ltd.) by binding a hydrochloric acid molecule to produce a Trazodone base, followed by reacting with succinic acid and crystallization of the product (Example 1).

For animal testing, compositions with various Trazodone Succinate contents were used in the form of tablets or drops for per os administration. The tablets were produced by direct compression in a number of variants containing different amounts of the active ingredient intended for different animal species and body weights (Example 1, Table 1). In the course of experiments, they were administered in an amount as required to achieve target doses (in mg/kg of an animal's weight). The liquid form was a Trazodone Succinate solution, further comprising one or two excipients selected from pharmaceutically acceptable stabilizers, preservatives, flavoring agents in amounts as required for achieving an effective dose of TR.

### b) Acute toxicity testing of Trazodone Succinate and a medicinal composition containing it

This parameter was evaluated on mice and rats in accordance with a conventional procedure for acute toxicity testing of medicinal products (Example 2). Based on the performed test results, the LD50 value was calculated for the Trazodone Succinate substance and a medicinal composition containing it, on the basis of which and the active ingredient, the resulting product was classified as a Hazard Class III substance according to the standard hygienic classification (GOST 12.1.007-76).

### c) Selecting criteria for determining the animal psychoemotional state and developing a system for assessment thereof.

Based on the data obtained by observing animals under stress, the most frequent symptoms of anxiety and fear common to various stressful situations were selected to assess the severity of their abnormal behavior. They included vocalizing, hypersalivation, overgrooming, abnormal (higher or lower) motion activity, soiling (urination and/or defecation), and 'destructive behavior' consisting in signs of aggression, attempts at damaging ambient objects, etc. For each symptom (based on its severity), points from 0 to 2 were given, which subsequently were totalized individually for each animal or, where appropriate, for a whole group of animals to determine an average value.

### d) Determining effective doses and side effect severity in case of using two TR salts.

Data regarding possible Trazodone Hydrochloride dosages for cats, as available from scientific literature, is very few and is eventually limited to information on the efficacy of a single dose administration of a 50 mg per capita (Stevens B., J Am Vet Med Assoc. 2016 249(2):202-7), which, for adult cats, corresponds to an average amount in a rather wide range from 6 to 25 mg/kg, and on administering higher doses (50 to 100 mg per capita) to produce a strong hypnotic action (Orlando JM., J Feline Med Surg. 2016, 18(6):476-82). This necessitated determining specific effective doses of the active ingredient (TR in the form of both salts: hydrochloride and succinate) within the above range. From comparative studies on cats with fear of transportation (Example 4, Tables VIa-VId) it was found that, to obtain the desired sedative effect with minimal side effect severity, the preferred active ingredient dose is 6±1 mg/kg. From comparison of the effects produced by the two different salts (hydrochloride and succinate), it apparently follows that where TR was used, at any studied doses, in the succinate form, the product produced much less intensive undesirable side effects in the form of general drowsiness, atony, slowness, dystaxia, than in case of using TR Hydrochloride, and, at the minimal of the studied doses, the side effects were totally absent.

According to the literature data, in dogs with various anxiety disorders, Trazodone in the hydrochloride form demonstrated acceptable levels of sedative action after a single dose administration at the following dosages: 3.5 to 12 mg/kg (Gilbert-Gregory, 2016), 7 to 10 mg/kg (Gruen, 2014); 5 mg/kg for dogs weighing more than 10 kg and 10 mg/kg for dogs weighing less than 10 kg (Orlando JM., J Feline Med Surg. 2016 Jun; 18(6):476-82).

In view of the availability of sufficiently specific (for various stresses) data on the dosages of Trazodone in the hydrochloride form for dogs, an active ingredient dose range from 5 to 10 mg/kg was pre-selected for further clinical trials on this animal species. Based on the experiment carried out by the inventors to adjust the dose for TR Succinate (Example 5), it was found that the dose preferred both in terms of efficacy and minimization of side effects corresponds to 6 ± 1 mg/kg.

### e) Clinical trials of Trazodone Succinate action in various stressful situations

The majority of the clinical studies (Example 6), as distinct from the experiment to compare the TR actions in the forms of two salts (the prior art salt and the salt of the present invention), included: a) administering the Trazodone Succinate-based product in a number of various stressful situations, b) in all cases, administering the product of the present invention in the same doses previously determined to be optimal to achieve the desired sedative effect with minimum side action severity (6 ± 1 mg/kg); c) to exclude the effect of habituation to the stimulus action, including into the test a control group of animals administered with a placebo product with external features and content of excipients similar to those of the test pieces, and d) using a value of the stressed state symptom severity averaged over a group to assess the psychoemotional state of the control and experimental animals.

Use of the novel product for modifying behavior in male and female cats during heat periods was studied in more detail (including assessment of individual behavior parameters) as part of the clinical trials (Example 7).

The obtained results demonstrated that the novel sedative product containing TR in the succinate form, when used for small domestic animals, is advantageous over the hydrochloride-based medicinal products in that it acts in a 'milder' manner with the same efficacy.

As such, it may be concluded, from the totality of the results so obtained, that a novel product has been developed on the basis of Trazodone Succinate for modifying behavior in small domestic animals under stressful conditions, enabling, within 30 to 60 minutes after administration, normalization (calming) of animal behavior in various stressful situations, including during a heat period, while not producing, at that time or subsequently, the undesirable side effects associated with the similar prior art medicinal products, such as drowsiness, atony, slowness or dystaxia (the technical result).

### Detailed Description of Embodiments

### Example 1. Producing Trazodone Succinate and a dosage form containing it

In the absence of a commercially available Trazodone Succinate substance, the salt was synthesized from Trazodone Hydrochloride (manufactured by Shenyang Funing Parmaceutical Co.Ltd.) by binding a hydrochloric acid molecule to produce a Trazodone base, followed by treating it with succinic acid. The refined substance was a white (or creamy white) crystalline powder with Tₘₑₗₜ. = 149-153°C, freely soluble in hot water, sparingly soluble in lukewarm water and alcohols, and chloroform-insoluble.

The structure of the compound so obtained was confirmed by the mass spectroscopy, NMR spectroscopy and elemental analysis methods at the Organic Microanalysis Laboratory at the Russian Academy of Sciences Institute of Organic Chemistry.

Trazodone Succinate effective doses were determined and clinical trials were carried out with the use of compounds with different active ingredient contents in the form of 100, 300 and 500 mg tablets and in the form of a solution for per os administration. The tablets were produced by direct compression in three variants containing different amounts of the active ingredient intended for different animal body weights (Table I). In the course of experiments, they were used in an amount as required to achieve target doses. Mannitol, silicon dioxide, magnesium stearate, and, optionally, a food grade dye, as are customary in this tableting method, were used as excipients. Due to this medicinal composition lacking any pronounced smell and having a pleasing slightly sour flavor, there was no need to add any components to mask or improve its taste.

**Table I. Variants of Tablets for Trials (containing green food grade dye).**

| Component | Component Content per Tablet (mg) | | |
|---|---|---|---|
| | 1 tablet (100 mg) per 4 kg of animal body weight | 1 tablet (300 mg) per 8 kg of animal body weight | 1 tablet (500 mg) per 20 kg of animal body weight |
| Trazodone Succinate | 24.0 | 48.0 | 120.0 |
| Mannitol | 71.9 | 147.9 | 375.9 |
| Silicon dioxide | 1.0 | 1.0 | 1.0 |
| Magnesium stearate | 3.0 | 3.0 | 3.0 |
| Food grade dye | 0.1 | 0.1 | 0.1 |

The product in the liquid form was produced by dissolving TR Succinate in hot water with beta- or hydroxypropyl -cyclodextrin (to increase solubility), followed by adding, into the solution so produced, a preservative (Nipagin, Nipasol, Sharomix) and, optionally, a flavoring additive. Preferably, 1 ml of the composition contained 50 mg of TR Succinate, 300 mg of hydroxypropyl -cyclodextrin and 0.01 ml of Sharomix. The tablet formulation was used in the majority of experiments, while the liquid form was used is certain trials (ref. to Example 4, Table 6d) in the 0.5 ml dose (25 mg of Tradozone Succinate)/4kg to compare its efficacy with that of the tablet formulation.

### Example 2. Acute oral toxicity testing of the substance (Trazodone Succinate) and the product

The tests were carried out at Stavropol State Agricultural University FGBOU VO. The experiments were made on mice and rats in accordance with a conventional procedure for acute toxicity testing of medicinal products, separately for the substance (Trazodone Succinatea) and the product containing it in the tablet form. In each case, 7 experimental groups and 1 control group of white rats and 7 experimental and 1 control groups of white mice, 10 individuals in each, were formed. The product was administered via an intragastric tube in accordance with the design described in Table I. 1% starch gel was used as the suspending agent.

**Table II. Design of Trazodone Succinate Substance Intragastric Administration to White Mice and Rats for Acute Toxicity Testing**

| Group # | Dose (mg/kg) | |
|---|---|---|
| | White Mice | White Rats |
| 1 | 400 | 400 |
| 2 | 800 | 800 |
| 3 | 1200 | 1600 |
| 4 | 1600 | 2300 |
| 5 | 2000 | 2700 |
| 6 | 2500 | 3000 |
| 7 | 3000 | 3500 |
| Control | Equal volume of 1% starch gel | |

Follow-up period was 14 days. The observation included general condition, behavior, feed and water intake, manifestation of intoxication symptoms, possible deaths.

Based on the test results, parameters of the substance acute toxicity were calculated according to Miller and Tainter (Tables III and IV).

**Table III. Trazodone Succinate LD50 Calculation According to Miller and Tainter**

| White Mice | | | | | |
|---|---|---|---|---|---|
| Laboratory Animal Groups | Dose (mg/kg) | Number of Animals | | % of deaths | Corresponding probits |
| | | survived | dead | | |
| 1 | 400 | 10 | 0 | 0 | 3.04 |
| 2 | 800 | 9 | 1 | 10.0 | 3.72 |
| 3 | 1200 | 5 | 5 | 50.0 | 5.00 |
| 4 | 1600 | 3 | 7 | 70.0 | 5.74 |
| 5 | 2000 | 0 | 10 | 100.0 | 6.96 |
| 6 | 2500 | 0 | 10 | 100.0 | 6.96 |
| 7 | 3000 | 0 | 10 | 100.0 | 6.96 |

| White Rats | | | | | |
|---|---|---|---|---|---|
| Laboratory Animal Groups | Dose (mg/kg) | Number of Animals | | % of deaths | Corresponding probits |
| | | survived | dead | | |
| 1 | 400 | 10 | 0 | 0 | 3.04 |
| 2 | 800 | 8 | 2 | 20.0 | 4.16 |
| 3 | 1600 | 6 | 4 | 40.0 | 4.75 |
| 4 | 2300 | 5 | 5 | 50.0 | 5.00 |
| 5 | 2700 | 3 | 7 | 70.0 | 5.75 |
| 6 | 3000 | 0 | 10 | 100.0 | 6.96 |
| 7 | 3500 | 0 | 10 | 100.0 | 6.96 |

**Table IV. Mice and Rat Acute Toxicity Metrics (mg/kg)**

| Species | Toxicity Metrics | | | | | SLD₅₀ |
|---|---|---|---|---|---|---|
| | MTD | LD₁₆ | LD₅₀ | LD₈₄ | LD₁₀₀ | |
| White Mice | 400 | 840 | 1200 (1091.6-1308.4) | 1690 | 1935 | ±108.4 |

| | Toxicity Metrics | | | | | SLD₅₀ |
|---|---|---|---|---|---|---|
| | MTD | LD₁₆ | LD₅₀ | LD₈₄ | LD₁₀₀ | |
| White Rats | 400 | 760 | 2300 (2079.5-2520.5) | 2730 | 2945 | ±220.5 |

From the data so obtained it follows that the median lethal doses of the Trazodone Succinate substance intragastrically administered to mice and rats were 1200 mg/kg and 2300 mg/kg, respectively.

Acute oral toxicity of the sedative product was tested in accordance with the same design, except that the animals were administered with a suspension of comminuted tablets in 1% starch gel.

LD50 values obtained for the sedative product were 1900mg/kg and 3400 mg/kg for mice and rats, respectively.

Thus, according to GOST 12.1.007-76, both the substance (Trazodone Succinate), and the tableted product containing it as an active ingredient are moderately hazardous substances (Hazard Class III).

### Example 3. Selection of criteria and a system for assessing the severity of abnormal behavior symptoms (psychoemotional state)

Based on the data obtained by observing animals under stress, the most frequent symptoms of anxiety and fear common to various stressful situations were selected to assess the severity of their abnormal behavior. They included 6 metrics: vocalizing, hypersalivation, overgrooming, abnormal (higher or lower) motion activity, soiling (urination and/or defecation), and 'destructive behavior' that includes such elements as aggressiveness, attempts at damaging ambient objects, etc. (Table V).

**Table V. Cat and Dog Psychoemotional State Assessment System**

| | Symptom | Score (points) | | |
|---|---|---|---|---|
| | | 2 | 1 | 0 |
| 1. | Vocalizing | Frequent/ periodic | Moderate | Natural or none |
| 2. | Motion activity | Excessive/ periodic | Motionlessness (behavioral depression) | Natural |
| 3. | Hypersalivation | Pronounced | Moderate | None |
| 4. | Grooming | Continuous/ frequent | Periodic | None |
| 5. | Soiling: urination and/or defecation | Pronounced | Moderate | None |
| 6. | Destructive behavior | Pronounced | Moderate | None |

For each of the 6 symptoms (based on its severity), points from 0 to 2 were given, which subsequently were totalized individually for each animal. Therein, a total score of more than 6 points (out of possible 12) was considered to be indicative of an apparent severity of abnormal (changed) behavior.

### Example 4. Comparison between the actions of Trazodone Hydrochloride and Succinate administered in the same doses

To carry out the experiment, 6 domestic cats of different sex and age, weighing 3.5 to 5 kg, were selected, who, according to their owners, felt bad during transportation, showing various forms of abnormal behavior. Herein, the actual presence of stress symptoms and their severity for each cat were assessed from video data provided by their owners.

The studies were carried out with the same animal group in two stages 1 month apart. At the first stage, the animals were administered, 1 hour prior to transportation and in the dose recommended in the literature, with the Trittico (Trazodone Hydrochloride) product at a rate of 50 mg of Trazodone per capita, and, a week later, with the novel product (containing Trazodone Succinate) in the same dose.

Each cat was transported from its place of residence for about 30 minutes in an individual pet carrier bag by its owner in a public or personal vehicle. The owners were accompanied on their trips by an investigator who was assessing the cats' psycho-emotional state (according to Table V) and recorded any symptoms of the medicinal product's side action on an individual record sheet. Furthermore, the pet owners had to record any particulars of the cats' behavior during the next few hours after returning home. The results obtained at the first stage of the experiment are given in Tables VIa 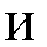 VIb.

**Table VIa. Assessment of Cats' Psychoemotional State during Transportation after Being Administered with Trittico (50 mg of active ingredient per capita).**

| # | Name | Breed, age, weight | Active ingredient dose (mg/kg) | Stressed state assessment (points) | | | | | Side effects during or after stress |
|---|---|---|---|---|---|---|---|---|---|
| | | | | Initially (*) | during transportation (minutes) | | | | |
| | | | | | 5 | 10 | 20 | 30 | |
| 1 | Vasya | N/P, 3 years, 4 kg | 12.5 | 6 | 0 | 1 | 0 | 0 | atony, dystaxia |
| 2 | Khryusha | N/P, 3 years, 4 kg | 12.5 | 8 | 7 | 7 | 5 | 4 | after the end of testing - heavy sleep during several hours |
| 3 | Vinny | N/P, 2 years, 4.5 kg | 11,1 | 9 | 1 | 0 | 1 | 1 | none |
| 4 | Flint | N/P, 2 years, 5 kg | 10.0 | 8 | 0 | 0 | 1 | 0 | drowsiness, dystaxia |
| 5 | Ksana | N/P, 11 years, 3.5 kg | 14.0 | 7 | 0 | 0 | 1 | 0 | drowsiness, lacrimation |
| 6 | Foks | N/P, 4 years, 5 kg | 10.0 | 7 | 4 | 4 | 4 | 3 | after the end of testing - heavy sleep during several hours |

| | | | | | | | | | |
|---|---|---|---|---|---|---|---|---|---|
| * Initial severity of fear symptoms in this trial was determined based on the owner interviewing and video records provided by them. | | | | | | | | | |

### Table VIb. Assessment of Cats' Psychoemotional State during Transportation after Being Administered with the Product of the Present Invention (48 mg of active ingredient per capita).

| # | Name | Breed, age, weight | Active ingredient dose (mg/kg) | Stressed state assessment (points) | | | | | Side effects during or after stress |
|---|---|---|---|---|---|---|---|---|---|
| | | | | initially | during transportation (minutes) | | | | |
| | | | | | 5 | 10 | 20 | 30 | |
| 1 | Vasya | N/P, 3 years, 4 kg | 12.0 | 6 | 0 | 0 | 0 | 0 | slight dystaxia |
| 2 | Khryusha | N/P, 3 years, 4 kg | 12.0 | 8 | 6 | 2 | 2 | 0 | none |
| 3 | Vinny | N/P, 2 years, 4.5 kg | 10.8 | 9 | 1 | 0 | 1 | 0 | none |
| 4 | Flint | N/P, 2 years, 5 kg | 9.6 | 8 | 2 | 0 | 0 | 0 | atony during 2-3 hours |
| 5 | Ksana | N/P, 11 years, 3.5 kg | 13.7 | 7 | 0 | 0 | 1 | 0 | none |
| 6 | Foks | N/P, 2 years, 4.5 kg | 9.6 | 7 | 2 | 2 | 0 | 0 | none |

From the data so obtained the following was concluded:
A) Both Trazodone Hydrochloride and Trazodone Succinate in a dose of 48 to 50 mg per capita (averaging 10 to 14 mg/kg) demonstrated, one hour after administration, pronounced sedative actions of comparable efficacy onto the animals.
B) TR Hydrochloride administration in the above dose caused pronounced side effects that consisted in atony and drowsiness, slow reactions and dystaxia in 5 out of the 6 subject animals (83.3%).

After the succinate administration, a side action was observed only in 2 out of the 6 cats (33.3%), which consisted in a certain degree of dystaxia and relatively short-time symptoms of atony.

The second stage of the experiment was carried out on the same animals one month after the first stage and in accordance with the same design, except that the doses of both products (Trittico containing Trazodone Hydrochloride and the novel product based on Trazodone Succinate) were reduced in half to 24-25 mg per capita or, in average, 5-7 mg/kg.

The results obtained at the second stage of the experiment are given in Tables VIc 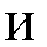 VId.

**Table VIc. Assessment of Cats' Psychoemotional State during Transportation after Being Administered with Trittico (25 mg of active ingredient per capita).**

| # | Name | Breed, age, weight | Active ingredient dose (mg/kg) | Stressed state assessment (points) | | | | | Side effects during or after stress |
|---|---|---|---|---|---|---|---|---|---|
| | | | | Initially (*) | during transportation (minutes) | | | | |
| | | | | | 5 | 10 | 20 | 30 | |
| 1 | Vasya | N/P, 3 years, 4 kg | 6.25 | 6 | 2 | 1 | 1 | 0 | none |
| 2 | Khryusha | N/P, 3 years, 4 kg | 6.25 | 8 | 6 | 3 | 3 | 2 | staggering |
| 3 | Vinny | N/P, 2 years, 4.5 kg | 5.6 | 9 | 5 | 2 | 0 | 0 | none |
| 4 | Flint | N/P, 2 years, 5 kg | 5.0 | 8 | 2 | 1 | 1 | 1 | general atony, drowsiness |
| 5 | Ksana | N/P, 11 years, 3.5 kg | 7.1 | 7 | 3 | 2 | 0 | 0 | drowsiness during 4-5 hours |
| 6 | Foks | N/P, 4 years, 5 kg | 5.0 | 7 | 4 | 3 | 2 | 2 | none |

**Table VId. Assessment of Cats' Psychoemotional State during Transportation after Being Administered with the Product of the Present Invention (24 mg of active ingredient per capita).**

| #, form | Name | Breed, age, weight | Active ingredient dose (mg/kg) | Stressed state assessment (points) | | | | | Side effects during or after stress |
|---|---|---|---|---|---|---|---|---|---|
| | | | | -Initially | during transportation (minutes) | | | | |
| | | | | | 5 | 10 | 20 | 30 | |
| 1*(1) | Vasya | N/P, 3 years, 4 kg | 6.0 | 6 | 2 | 0 | 0 | 0 | none |
| 2 | Khryusha | N/P, 3 years, 4 kg | 6.0 | 8 | 3 | 3 | 1 | 0 | none |
| 3* (1) | Vinny | N/P, 2 years, 4.5 kg | 5.3 | 9 | 2 | 0 | 1 | 0 | none |
| 4 | Flint | N/P, 2 years, 5 kg | 4.8 | 8 | 1 | 1 | 0 | 0 | none |
| 5* (1) | Ksana | N/P, 11 years, 3.5 kg | 6.9 | 7 | 0 | 0 | 1 | 0 | slight slowness in reactions |
| 6 | Foks | N/P, 4 years, 5 kg | 4.8 | 7 | 4 | 3 | 3 | 1 | none |

| | | | | | | | | | |
|---|---|---|---|---|---|---|---|---|---|
| *Note:* (1)* - the product was administered in the liquid form. | | | | | | | | | |

From an analysis of the data so obtained, the following conclusions could be drawn:
a) Where the products were administered in doses ranging from 5 to 7 mg/kg, Trazodone Hydrochloride acted slightly slower than Trazodone Succinate in at least half the cats in response to a stressful situation occurrence.
b) Side effects in the cats administered with the product containing TR Hydrochloride, though considerably less intensive than at the first stage (with higher active ingredient doses), were recorded in 3 out of the 6 animals, while where TR Succinate were administered, only one case of a slight slowness in reactions to external actions (in an elder female cate with a low weight) was reported.
c) Administration to half the cats who were administered with Trazodone Succinate in a therapeutically effective dose (5-7 mg/kg) of a liquid dosage form instead of a solid dosage form (with the same active ingredient concentration) did not cause any noticeable changes neither in side effects, nor in the efficacy of action, except that a somewhat earlier onset of the sedative effect could be visually observed in the cats administered with the product in the liquid form. This fact can be adequately explained by the presence of cyclodextrin which is known for its ability to enhance solubility and bioavailability of medicinal products (RU 2419621 C2, 27.05.2011; Yang GF et al., 2006, J.Phys. Chem. B, 110 (13), 7044-8, etc.) and may be given further consideration in terms of further reducing the therapeutic dose for the novel product in such form.

Thus, the comparative experiment so performed has demonstrated that the side action is dose-dependent; however, with the equal active ingredient doses, either low, or high, it is considerably less intensive, both quantitatively and qualitatively, in the cases where RT is administered in the succinate form, than in those where it is administered in the hydrochloride form. Furthermore, it was found that, where the dose was reduced in average to 6±1.0 mg/kg in case of the Trittico tablets (containing the hydrochloride), the percentage of animals with side effects remained high (50%), but the timing of the sedative effect onset was somewhat slower, while the novel product (containing TR Succinate) has demonstrated an action as efficient as with higher doses, but eventually without any side effects. The dose of 6±1 mg/kg has been selected for further clinical trials in cats of the novel product containing TR Succinate.

### Example 5. Adjusting the Trazodone Succinate dose for dogs.

The experiment has been carried out in the separation anxiety conditions with doses in the range which, according to the literature data, is considered to provide the desired sedative effect in various stressful situations in cases where TR-containing commercially available products are used and is about 5 to 10 mg/kg.

Enrolled in the test were domestic dogs feeling anxiety when separated from their owners (to support the anamnesis, the owners provided video records of the animals in periods of separation).

Each of the dogs was orally administered with a sedative product, wherein three of them were given the dose (of Trazodone Succinate) of 9 mg/kg and the other three were given the dose of 6 mg/kg. After the product administration, the owners left their dogs who were placed, for 4 hours, in individual cages in a room at the inpatient facility of Bio-Vet Veterinary Clinic.

The animal observation data was recorded on an hourly basis. The sedative product efficacy was determined in accordance with a pre-developed assessment scheme (Table V). Close attention was paid to the medicinal product's side actions that were recorded in a dedicated section of an assessment sheet. Upon completion of the experiment, the owners were requested to record any peculiarities of their pets' behavior during further 4-6 hours at home.

The 6 doges enrolled in the experiment shown the separation anxiety symptoms consisting in vocalizing (2 points - all dogs), increased motion activity (2 points - all dogs), destructive behavior (Dogs 4 and 5), overgrooming (Dogs 1, 2, 3 and 5), soiling (Dogs 1 and 6). The total score for the animals enrolled in the experiment was 7 to 10 points out of 12 possible points, which is indicative of an apparent severity of the separation anxiety in the subject animals.

The study results are given in Table VII.

**Table VII. Assessment of Psychoemotional State of Dogs Anxious due to Separation from their Owners after Administration of the Product of the Present Invention**

| # | Name | Breed, Age, Weight | Active ingredient dose (mg/ kg) | Assessment of the state at the time of selection (points) | Assessment of the state after separation from the owner (over time, hours), points | | | | | Side actions |
|---|---|---|---|---|---|---|---|---|---|---|
| | | | | | 0.5 | 1 | 2 | 3 | 4 | |
| 1 | Dany | Toy Terrier, 3 years, 4 kg | 9 | 8 | 8 | 3 | 0 | 0 | 0 | certain slowness in reactions during the experiment and 2- 3 hours thereafter |
| 2 | Nyusha | N/P, 4 years, 20 kg | 9 | 8 | 6 | 0 | 0 | 0 | 0 | drowsiness during 6-7 hours |
| 3 | Liza | N/P, 13 years, 10 kg | 9 | 10 | 8 | 0 | 0 | 0 | 0 | none |
| 4 | Deiny | Yorkshire Terrier, 2 years, 4 kg | 6 | 7 | 7 | 3 | 0 | 0 | 0 | none |
| 5 | Kuzya | Basenji, 8 years, 12 kg | 6 | 7 | 7 | 0 | 0 | 0 | 0 | none |
| 6 | Ksyusha | Toy Terrier, 1.5 years, 3 kg | 6 | 8 | 6 | 0 | 0 | 0 | 0 | none |

As can be seen from the data given in Table VII, an apparent sedative effect was observed in all the dogs, which consisted, in each animal, either in a total disappearance of the major fear symptoms or in a significant reduction in their intensity. A sustained sedative action occurred 1 hour after the product administration and separation from the owner.

With an apparent therapeutic effect, the Trazodone dose of 9 mg/kg caused a side action consisting in slight slowness in one of the dogs and a long-time state of drowsiness in another dog. With the TR dose of 6 mg/kg, the product did not cause any side action in any of the dogs. As such, the sedative product of the present invention was used in the dose (of Trazodone) of 6±1 mg/kg in subsequent clinical studies on dogs, i.e. in the same therapeutic doses as for cats.

### Example 6. Clinical trials of the Trazodone Succinate-containing product in various stressful situations associated with fear or anxiety

Clinical studies of the product for modifying abnormal behavior in small domestic animals of the present invention were carried out in Moscow and Moscow Oblast; some experiments were set up on the basis of BioVet Veterinary Clinic (Moscow). The systemic clinical studies, as distinct from the two assessment experiments to compare the action of TR in the form of two salts (the prior art and that of the present invention) (Example 4) and to adjust the active ingredient dose for dogs (Example 5), included:
a) administering the Trazodone Succinate-based product in a number of various stressful situations associated with different symptoms of fear or anxiety which included (with due account of the scope of the studies carried out at the first stage of the comparative trials regarding the fear of transportation in cats and separation anxiety in dogs) the following: fear of transportation in dogs, separation anxiety in cats, and fear of loud sounds in cats and dogs;
b) administering the product of the present invention in the same dosage of Trazodone Succinate, which was previously determined to be optimal for achieving the desired sedative effect with minimum manifestation of any side action (6± 1 mg/kg);
c) to exclude the effect of habituation to the stimulus action, the treatment efficacy was determined in comparison with a control group of animals administered with a placebo product with external features and content of excipients similar to those of the test pieces; and
d) using a value of the stressed state symptom severity averaged over a group to assess the psychoemotional state of the control and experimental animals.

### Animal selection and general experimental design

The animals were selected from among adult, apparently healthy, cats and dogs living at home, who, according to their owners, felt fear (anxiety) for certain stimuli or situations. Prior to the trials, preliminary experiments had been made, which included observing the animal behavior in a situation reported by the owner, and only those animals were selected, for whom the preliminary anamnesis was confirmed with a high severity of the fear (or anxiety) symptoms according to the system of psychoemotional state assessment (in points) developed by us and presented in Table V (Example 3).

Typically, 12 individuals were enrolled in an experiment, from whom 2 groups were randomly (based on a table produced using a random number generator, available at: *https:*//*randstuff.ru*/*number*/*)* formed: an experimental group and a control group (6 animals in each), wherein the animals in the first group were exposed to a stimulus after having been administered with the medicinal product (Trazodone Succinate), while those of the second group were exposed to the stimulus after having been administered with a placebo product containing only the excipients included into the subject sedative product. The animals were administered with a product (the experimental ones - with TR Succinate in the amount providing the predetermined optimum dose, and control ones - with the placebo) as a single dose, before feeding, orally, forcedly (by placing on the tongue root or using a tablet introducer), typically one hour prior to exposure to the stress factor, followed by continuously observing the animals by an investigation officer. The observation data was recorded on individual sheets, and then each animal's psychoemotional state was assessed in points and an average fear symptom severity value was determined for the entire group (the experimental or the control one). The data so obtained was statistically processed using the Microsoft Excel application. In addition to the product efficacy, observation data was entered into a dedicated column of an animal's individual sheet, regarding the sedative product's side effect manifestation, if any.

### Fear of Transportation (dogs).

Animals for the experiment had been initially selected from among adult, apparently healthy dogs of different sex and breed, who, according to their owners, felt anxiety when separated from their owners. To verify the anamnesis, a specialist accompanied an owner with his/her animal on a transport trip and recorded (including an assessment in point as per Table V) any manifestation of fear symptoms when the product was not used. Based on the verification results, 12 animals were enrolled in the experiment (8 males and 4 females) aged 2 to 5 years, weighing 2 to 50 kg, with a fear symptom severity of more than 6 points, from whom a control and an experimental groups were subsequently formed by the method described above.

Transportation in a public or a personal vehicle from a place of residence lasted about 30 minutes first one hour, and then 3.6 and 8 hours, after the product administration. The owners were accompanied on their trips by an investigator who was recording the dogs' psycho-emotional state data as per Table V on individual record sheets.

Small-breed dogs were transported in individual pet carrier bags, while medium- and large-breed dogs were on leash and wearing muzzles.

The results of determining the product efficacy in correcting dog behavior issues associated with transportation are given in Table VIII.

**Table VIII. Assessment of transportation fear severity in dogs**

| Experiment duration (hours) | Average score of stress symptom severity in a group | |
|---|---|---|
| | | |
| 0 | 7.33±0.33 | 7.83±0.17 |
| 1 | 1.5±0.43 | 7.0±0.26 |
| 3 | 1.33±0.21 | 6.83±0.31 |
| 6 | 2.83±0.60 | 7±0.26 |
| 8 | 5.33±0.33 | 7.17±0.31 |

As can be seen from the above data, a sedative action was observed in all dogs of the experimental group transported 1 and 3 hours after the product administration, which action, though incomplete, was well pronounced and sufficient to carry out the transportation (a fear symptom severity score averaged over the group was 1.3 to 1.5), and, though in a less pronounced degree (with an average score of 2.8), was registered after 6 hours and eventually disappeared only after 8 hours (with an average score of 5.3). All control animals demonstrated symptoms of fear during the 4 trips with equal (their initial) degree of severity, which is indicative of the absence of stress 'habituation' during the experiment.

No side effects were observed in the animals administered with the product either during the experiment, or after returning home.

### Separation anxiety (cats)

Animals for the experiment had been selected from among adult, apparently healthy cats of different sex and breed, living at home, who, according to their owners, felt anxiety when separated from their owners. To verify the presence of this behavioral issue, the animals were observed directly in the ambient conditions they were kept in, both in the presence and in the absence of their owners (without being administered with the product). Based on the observation results, 12 animals were enrolled in the study (8 males and 4 females), aged 2 to 8 years, weighing 3 to 5 kg, with the most pronounced (scoring more than 6 out of 12 points) manifestations of anxiety symptoms (vocalizing, motion activity, destructive behavior and soiling).

The study was carried out in domiciliary conditions in accordance with the following design:
a) The product and the placebo were administered before feeding, orally, forcedly, as a single dose.
b) After the animal had been administered with the product, the owner in the absence of whom the cat felt the separation anxiety, left the flat and was absent for at least 8 hours. The animal was observed by the rest of the family staying at home, who recorded (at predetermined time intervals), on a dedicated assessment sheet, the occurrence and degree of severity of such symptoms as defecation and urination outside its litter box, destructive behavior, vocalizing, inappetency, grooming or cleaning itself (from hair).
c) Upon completion of the experiment, the sedative product efficacy was assessed based on the data obtained by observing the animal during 8 hours and presented in the form of points as per Table V).

The data so obtained are given in Table IX.

**Table IX. Assessment of Separation Anxiety Symptom Severity in Cats**

| # | Name | Assessment of the state at the time of selection (points) | Assessment of the state after separation from the owner (after a predetermine time, hours) (points) | | | | | | | | |
|---|---|---|---|---|---|---|---|---|---|---|---|
| | | | 0.5h | 1 h | 2h | 3 h | 4h | 5 h | 6 h | 7h | 8h |
| | **Experimental group** | | | | | | | | | | |
| 1 | Murka | 6 | 7 | 2 | 1 | 1 | 2 | 1 | 2 | 3 | 7 |
| 2 | Pyata | 8 | 7 | 2 | 1 | 0 | 1 | 0 | 0 | 2 | 4 |
| 3 | Taiga | 8 | 8 | 3 | 3 | 2 | 0 | 0 | 0 | 3 | 4 |
| 4 | Kot | 7 | 6 | 1 | 0 | 1 | 1 | 1 | 1 | 2 | 6 |
| 5 | Mysh | 8 | 5 | 5 | 1 | 0 | 1 | 1 | 0 | 2 | 4 |
| 6 | Fenya | 9 | 6 | 4 | 1 | 0 | 1 | 1 | 2 | 8 | 6 |
| | average | 7.67 | 6.5 | 2.83 | 1.67 | 0.67 | 1.0 | 0.67 | 0.83 | 3.33 | 5.17 |

| | **Control group** | | | | | | | | | | |
|---|---|---|---|---|---|---|---|---|---|---|---|
| 7 | Pups | 7 | 6 | 6 | 8 | 6 | 6 | 6 | 4 | 5 | 6 |
| 8 | Nastya | 7 | 6 | 6 | 7 | 6 | 6 | 9 | 6 | 7 | 7 |
| 9 | Marusya | 9 | 7 | 7 | 5 | 7 | 7 | 7 | 6 | 5 | 5 |
| 10 | Dusya | 7 | 7 | 6 | 6 | 7 | 6 | 5 | 4 | 6 | 6 |
| 11 | Konyok | 8 | 6 | 7 | 7 | 6 | 6 | 6 | 6 | 6 | 8 |
| 12 | Norka | 7 | 7 | 7 | 8 | 8 | 6 | 7 | 5 | 7 | 6 |
| | average | 7.5 | 6.5 | 6.5 | 6.67 | 7.33 | 6.5 | 7.0 | 5.83 | 6.33 | 6.33 |

In as little as 60 minutes after the product administration, the observer recorded a considerable sedative action (2.83±0.60 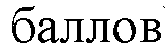 ) consisting in apparent mitigation of typically manifested symptoms of anxiety. The animals were calm and played.

After 3 hours from the product administration, its therapeutic action not only remained, but also increased (per group): the anxiety symptoms completely disappeared in all the cats (the group's average score = 0.67±0.33). After 6 hours of being separated from their owners, it was observed that anxiety in the cats was gradually increasing; after 7 hours from the product administration, the group's average score was as much as 3.33±0.95 points, and after 8 hours it eventually returned to a level close to the initial one of 5.17±0.54.

No side effects (such as, sedative effect, atony, slowness in reactions, dystaxia) were observed.

### Fear of loud sounds (cats)

The study enrolled 12 cats with the most pronounced manifestations of fear of loud sounds, including males (3) and females (9) of different breed, weighing 3.5 to 6 kg, aged 4 to 9 years, randomly divided into 2 groups: an experimental group and a control group.

After administration of the medicinal product (or a placebo product), the animals were placed in individual cages arranged in a veterinary clinic's inpatient facility and left there for the next 8 hours. A wireless portable acoustic system (BBK BTA7000) was installed behind a panel screen in front of a cage (at a 1-m distance from it) to play back a record of firecracker sounds at a magnitude of 80 dB. The playback was turned on after 1, 3, 6 and 8 hours after the product administration and lasted at least 1 minute.

By the first action of the stimulus (60 minutes after the product administration), the animals were calm and eventually not responded to the repeated (3 and 6 hours later) exposure to the stimulus (Table X).

**Table X. Assessment of loud sound fear severity in cats**

| Experiment durati on (hours) | Average group score of stress symptom severity | |
|---|---|---|
| | Experimental group | Control group |
| 0 | 5.5±0.22 | 4.83±0.17 |
| 1 | 0.3±0.21 | 4.33±0.33 |
| 3 | 1.33±0.21 | 4.67±0.21 |
| 6 | 1.83±0.31 | 4.0±0.37 |
| 8 | 4.83±0.40 | 4.50±0.52 |

The average score (in points) of assessed experimental cats' response to the stimulus after one hour was close to 0. The experimental animals' calm response to the loud sound (a repeated exposure to the stimulus 3 hours after the product administration) remained. After 6 hours from the product administration, a gradual recurrence of the fear symptoms was recorded in the experimental cats (the average group score was 1.8), and after 8 hours the average severity score was close to the initial value (4.83±0.40 points). High severity of the fear symptoms in the control group cats remained at the level close to the initial one throughout the experiment.

No side effects, such as dystaxia, weakness, slowness, or heavy sleep, were recorded in the cats administered with the product.

### Dogs

12 apparently health animals (with a loud sound fear severity of more than 6 points) were selected for the studies, including males (6) and females (6) of different breed, weighing 1.0 to 55 kg, aged 2 to 12 years. The selected animals were divided into 2 groups: an experimental group and a control group, 6 animals in each.

The results of the performed trials are given in Table XI.

**Table XI. Assessment of loud sound fear severity in dogs**

| Experiment duration (hours) | Average group score of stress symptom severity | |
|---|---|---|
| | Experimental group | Control group |
| 0 | 6.33±0.33 | 6.17±0.33 |
| 1 | 0.5±0.22 | 6.17±0.31 |
| 3 | 1.5±0.22 | 6.0±0.26 |
| 6 | 2.67±0.33 | 6.0±0.33 |
| 8 | 5.67±0.21 | 6.0±0.30 |

It can be concluded from the results given in Table XI that all dogs in the experimental group, in contrast to those in the control group, as early as 1 hour after the product administration, responded to the stimulus action eventually without any pronounced symptom of fear (average group score of 0.5).

The product's sedative action, though to a smaller degree than during the first 3 hours (average score of 1.5), was observed during 6 hours (average group score of 2.67). After 8 hours the average score of fear severity came close to the initial value (5.83 points). However, no side effects, including those associated with Trazodone Hydrochloride, were observed in any of the experimental animals.

No signs of adaptation or 'habituation' to the acoustic signal were observed in the control group when the stimulus actions were repeated: the fear severity remained at the initial level (an average score was 6.17 to 6.0 points).

### Example 7. Assessment of Trazodone Succinate potential as an express method for suppressing behavioral signs of heat

The trials enrolled apparently healthy entire animals (8 male cats and 8 female cats) aged 2 to 6 years, weighing 3 to 4 kg, kept in foster care at BIM Animal Shelter. The females were introduced into the study as they went into estrus.

**Table XII. Registration details of the cats enrolled in the experiment**

| # | Chip ID | Sex | Breed | Weight (g) | Age |
|---|---|---|---|---|---|
| 1 | 8222 | ♂ | Non pedigree | 3600 | 8 |
| 2 | 7202 | ♂ | Non pedigree | 3200 | 6 |
| 3 | 7223 | ♂ | Non pedigree | 4100 | 4 |
| 4 | 6933 | ♂ | Non pedigree | 3900 | 2 |
| 5 | 6477 | ♂ | Non pedigree | 4100 | 5 |
| 6 | 0559 | ♂ | Non pedigree | 3800 | 6 |
| 7 | 0982 | ♂ | Non pedigree | 3900 | 4 |
| 8 | 3245 | ♂ | Non pedigree | 4000 | 6 |
| 9 | 3845 | ♀ | Non pedigree | 4100 | 4 |
| 10 | 2942 | ♀ | Non pedigree | 2800 | 5 |
| 11 | 1317 | ♀ | Non pedigree | 3900 | 5 |
| 12 | 1318 | ♀ | Non pedigree | 4000 | 3 |
| 13 | 7512 | ♀ | Persian | 2800 | 2 |
| 14 | 5389 | ♀ | Non pedigree | 3700 | 3 |
| 15 | 7417 | ♀ | Non pedigree | 3900 | 3 |
| 16 | 7474 | ♀ | Non pedigree | 2900 | 5 |

### Experiment design for female cats

Female cats with pronounced signs of heat were placed in individual cages in a separate room. At least two entire male cats were kept in cages in the same room. In the morning, before feeding, the female cats were orally forcedly administered with a tablet (100mg) of either the product (experimental animals), or of placebo (control animals). Observation of animals' behavior began 30 minutes after the product administration and was carried out during 8 hours; the female cats' psychoemotional state details (in points) and side actions were recorded on individual sheets.

### Experiment design for male cats

Male cats with pronounced signs of heat were placed in individual cages in a dedicated room where 2 female cats in heat were present (in separate cages in the same room). TR Succinate in the tablet form (for the experimental animals) and placebo tablets (for the control animals) were given in accordance with the same design as in the experiment with female cats. Follow-up observation over the animals was carried out in a similar manner. The trial results are given in Tables XIII and XIV.

**Table XIII. Assessment of the intensity of sexual behavior in female cats before and after Trazodone Succinate administration**

| Chip ID | Total intensity of heat signs before the product administration (points) | Total intensity of heat signs after the product administration (points) | | | | |
|---|---|---|---|---|---|---|
| | | after 30 minutes | after 1 hour | after 3 hours | after 6 hours | after 8 hours |
| **Experimental group** | | | | | | |
| 3845 | 7 | 5 | 0 | 1 | 2 | 4 |
| 2942 | 8 | 6 | 0 | 1 | 1 | 1 |
| 1317 | 8 | 4 | 0 | 1 | 1 | 1 |
| 1318 | 8 | 4 | 1 | 2 | 2 | 5 |
| 7512 | 8 | 5 | 1 | 1 | 2 | 4 |
| 5389 | 7 | 5 | 0 | 1 | 2 | 1 |

| **Control group** | | | | | | |
|---|---|---|---|---|---|---|
| 7417 | 7 | 6 | 5 | 6 | 7 | 6 |
| 7474 | 8 | 6 | 7 | 6 | 5 | 7 |

As can be seen from the above Table XIII, a noticeable reduction in the heat sign intensity was observed in all cats of the experimental group as early as after 30 minutes after the product administration; after 1 hour, a substantially complete calmness was observed in the animals, which consisted in disappearing or maximum mitigation of such estrous behavior signs as vocalizing, hyperactivity, lordosis, etc., and remained for at least during 6 hours. After 8 hours, a gradual recurrence of the typical sexual behavior signs was observed in half the female cats, which, however, were less intensive than before the sedative product administration. The initial level of the heat sign intensity remained in the female cats administered with the placebo throughout the observation period.

No side effects, such as pronounced hypnotic action, atony, slowness or dystaxia, were observed in the experimental group animals during the experiment.

**Table XIV. Assessment of the intensity of sexual behavior in male cats before and after Trazodone Succinate administration**

| Chip ID | Total intensity of heat signs before the product administration (points) | Total intensity of heat signs after the product administration (points) | | | | |
|---|---|---|---|---|---|---|
| | | after 30 minutes | after 1 hour | after 3 hours | after 6 hours | after 8 hours |
| **Experimental group** | | | | | | |
| 8222 | 5 | 5 | 0 | 1 | 2 | 4 |
| 7202 | 6 | 6 | 0 | 1 | 1 | 4 |
| 7223 | 6 | 4 | 0 | 2 | 1 | 1 |
| 6933 | 5 | 4 | 1 | 2 | 2 | 3 |
| 6477 | 6 | 5 | 1 | 1 | 2 | 1 |
| 0559 | 8 | 5 | 0 | 1 | 2 | 4 |

| **Control group** | | | | | | |
|---|---|---|---|---|---|---|
| 7417 | 6 | 6 | 4 | 6 | 7 | 6 |
| 7474 | 7 | 7 | 5 | 6 | 6 | 6 |

As is apparent from the data in the above Table, in all male cats administered with the product (Trazodone Succinate), a substantially complete suppression of the undesirable signs of sexual behavior occurred within 30 to 60 minutes after the product administration. A high level of Trazodone Succinate sedative action remained until after 6 hours from the product administration; after 8 hours, the maximum level of the product's sedative effect was still observed in only 2 out of 6 animals. Same as in the experiment on female cats, no abnormal behaviors were observed in the animals.

As such, a single-dose administration of Trazodone Succinate in a dose of 6-8 mg/kg (1 table containing 24 mg of the active ingredient for an animal weighing 3 to 4 kg) produced a pronounced sedative action in female and male cats in heat, which started 30 minutes after the product administration, reached its maximum (substantially complete disappearance of estrous behavior signs) after 1 hour and remained for at least 6 hours (typically 7 to 8 hours).

During the time course of the product, the animals were calm, but not in heavy sleep, and adequately responded to what was going around them. No side actions, such as dystaxia, slowness or other undesirable reactions, were observed.

### Industrial Applicability

In summary, it may be concluded that, as a result of the present invention, a novel product has been developed, which may be widely used in veterinary practice for cats and dogs and allows efficiently addressing the problem of modifying abnormal behavior in animals, which is associated with either a stress caused by various external factors (transportation, loud sounds, separation from the owner, fear of new locations, etc.), or the heat period. The product of the present invention is the first veterinary Trazodone-containing product for said application, wherein TR, as the active ingredient, is used in the form of succinic acid salt (succinate). Trazodone Succinate in the medicinal product of the present invention enables a complete elimination, without reducing the product efficacy, or at least substantial suppression of the major side effects reported for all medicinal products containing TR, in which it is typically used in a free state or in the hydrochloride form.

## Claims

1. A pharmaceutical composition for correcting the behaviour of cats and dogs in stressful situations **characterized in that** the composition comprises as an active principle Trazodone Succinate in a concentration providing an effective dose in a single-dose administration and pharmaceutically acceptable filles.

2. The pharmaceutical composition of Claim 1 **characterized in that** it is administered in a dose of 5 to 14 mg/kg animal weight.

3. The pharmaceutical composition of Claim 2 **characterized in that** it is administered in a dose of 6±1 mg/kg animal weight.

4. The pharmaceutical composition of Claim 1 **characterized in that** it is configured in a tablet form.

5. The pharmaceutical composition of Claim 1 **characterized in that** it is configured in a liquid form.
